# EUROPEAN PATENT SPECIFICATION

(11) **EP 2 222 178 B1**
(45) Date of publication and mention of the grant of the patent: **02.05.2018**
(21) Application number: 08865466.0
(22) Date of filing: 08.12.2008
(51) Int. Cl.: A23C 19/032, A23C 19/068

(54) **ACCELERATED FUNGAL GROWTH**
BESCHLEUNIGTES PILZWACHSTUM
DÉVELOPPEMENT FONGIQUE ACCÉLÉRÉ

(30) Priority: 20.12.2007 EP 07123810
(43) Date of publication of application: 01.09.2010
(60) Divisional application: 18163879.2
(73) Proprietor: DSM IP Assets B.V., 6411 TE Heerlen (NL)
(72) Inventor: SAVAGE, Robert John Bromley, Llangollen LL20 7PE (GB); ROOIJEN, VAN, Rutger Jan, NL-6713 MN Ede (NL); DIJK, VAN Albertus, Alard, NL-2600 MA Delft (NL)
(74) Representative: DSM Intellectual Property
(86) International application number: PCT/EP2008/067031
(87) International publication number: WO 2009/080484

(56) References cited:
- WO-A-01/30172
- WO-A-2005/074695
- WO-A-2005/117595
- WO-A-2006/087350
- WO-A-2007/060247
- JP-A- 2000 270 758
- DATABASE WPI Week 198306 Thomson Scientific, London, GB; AN 1983-13016K XP002480272 & JP 57 208988 A (SNOW BRAND MILK PROD CO LTD) 22 December 1982 (1982-12-22)
- GRIPON ET AL: "The proteolytic system of Penicillium roqueforti" BIOCHIMIE, MASSON, PARIS, FR, vol. 59, no. 8-9, 28 November 1977 (1977-11-28), pages 679-686, XP022092689 ISSN: 0300-9084
- BOUTROU ET AL: "Enhanced proteolytic activities of Geotrichum candidum and Penicillium camembertii in mixed culture" ENZYME AND MICROBIAL TECHNOLOGY, STONEHAM, MA, US, vol. 39, no. 2, 26 June 2006 (2006-06-26), pages 325-331, XP005434051 ISSN: 0141-0229
- GÓMEZ ET AL.: "Enzyme Activity in Spanish Goat's Cheeses" FOOD CHEMISTRY, vol. 28, no. 2, 1988, pages 159-165, XP002480271

## Description

### Field of the invention

The present invention relates to a method for accelerating fungal growth. In particular, it relates to the acceleration of fungal growth in fermented food.

### Background of the invention

Flavour of food products is one of the key attributes for the consumer. In fermented products, e.g. dairy products, flavors are derived from milk components by enzymatic activities of micro-organisms. In cheese for instance, various flavour compounds have been identified as being essential and many of them are derived from casein degradation. Other enzymatic processes, such as lipolysis, are also involved, most notably in cheese where fungi are involved in the ripening process, *e.g*. Camembert and Roquefort cheese. In addition, lactose fermentation might lead to the flavour compounds such as propionic acid (Smit et al, Food Res. Int. (2000) 33, 153-160).

Proteolysis in cheese during ripening plays a vital role in the development of texture as well as flavour and has been subject of several reviews (see *e.g*. McSweeney & Sousa, Lait (2000) 80, 293-324). Proteolysis contributes to textural changes of the cheese matrix, due to breakdown of the protein network, decrease in *a_{w}*, through water binding by liberated carboxyl and amino groups and increase in pH, which facilitates the release of sapid compounds during mastication (Sousa et al, Int. Dairy Journal (2001), 11, 327-345). It contributes directly to flavour and to off-flavour (*e.g.* bitterness) of cheese through the formation of peptides and free amino acids as well as liberation of substrates (amino acids) for secondary catabolic changes, *i.e*. transamination, deamination, decarboxylation, desulphuration, catabolism of aromatic amino acids and reactions of amino acids with other compounds. The rate and pattern of proteolysis may be influenced by location within the cheese.

Cheese ripening is a time-consuming process involving complex and well-balanced reactions between glycolysis, proteolysis and lipolysis of the milk components. In most cheeses, bacterial enzymes play a major role in this process. It is well known that changing the bacterial enzyme content in cheese directly affects the rate of cheese ripening and its final flavour (Klein & Lortal, Int. Dairy Journal (1999) 9, 751-762.). A way to influence cheese ripening is to increase the bacterial enzyme pool in cheese curd by the addition of whole lactic acid bacteria, unable to grow and produce significant levels of lactic acid, but still delivering active ripening enzymes during cheese ageing. The starters are normally weakened and referred to as attenuated.

Since cheese ripening is a time consuming process it is also costly. Cheeses need to be stored during ripening under precisely defined conditions for temperature and humidity for weeks to months. The ripening time varies considerably between the various cheeses, from 3 weeks (*e.g*. Mozzarella) to more than 2 years (*e.g*. Parmesan, extra mature cheddar). Any process that would result in acceleration of cheese ripening is interesting from an economic point of view: the same amount of cheese can be produced in a shorter time interval.

Proteolysis in cheese is a very complex process, and proteases from various origins are involved (for review see e.g. Fox & McSweeney, Food Rev. Int (1996) 12, 457-509). Such proteases are the coagulant that was used during cheese manufacture (e.g. chymosin, pepsin or fungal acid proteinases), milk own proteins (e.g. plasmin), the proteases provided by the starter bacteria, proteases from non-starter adventitious microflora, proteases from a second inocculum (in some varieties, *e.g. P. roqueforti, P camemberti, Br. Linens*), attenuated bacterial cells and exogenous proteases. The attenuated cells and the exogenous proteases are recent tools in the development of acceleration of cheese ripening. The generation of free amino acids is an important step in the acceleration of cheese ripening. Although the free amino acids contribute to the overall cheese flavour, their contribution is relatively small. The amino acids are the precursors, which are subsequently converted by the micro-organisms that are present in the cheese to flavour compounds. Availability of amino acids is therefore important for cheese flavour formation and thus for cheese ripening.

Many proteases are involved in the generation of cheese flavours. The generation of the proper flavour for a cheese requires a delicate balance of proteolytic activities from the proteases involved. Any imbalance will easily lead to flavours that are not wanted, such as bitterness development. Especially the development of bitterness in cheese has been well described and documented (see *e.g*. LeMieux & Simard, Lait (1991) 71, 599-636; Lemieux & Simard, Lait (1992) 72, 335-382). In cheeses that use a combination of bacterial and fungal species to obtain a particular flavour profile the situation is particularly complicated. In e.g. the production of Blue Stilton, the bacterial microflora is used in the initial stages of cheese making to obtain curd and initial flavour formation. In a second stage, the cheese is pierced with needles to allow air in the interior of the cheese matrix. This allows the germination of the fungal spores that were introduced in the cheese during the making process. General manufacturing considerations for blue cheeses, including blue veined cheeses such as Stilton, are described (see *e.g*. J.C. Gripon in Encyclopaedia of dairy Sciences (2003, Roginsky et al, eds, Academic Press), pp 410-406). The fungus produces a complex enzyme mixture, including lipases and proteases, which are important for growth and flavour development, as well as for the generation of a creamy, indulgent texture. Excessive growth of the fungus results in bitterness formation resulting from the formation of bitter peptides (J.C. Gripon in Encyclopaedia of Dairy Sciences (2003), Roginsky et al, eds, Academic Press, pp 410-406).

The development of proteases for cheese to enhance ripening processes is therefore a very delicate and complicated process. Exo-proteases are preferred over endo-proteases because they have a lower tendency to induce formation of bitterness. Endo-proteases are known to easily introduce such bitterness and are therefore preferably not used. There is, however, a clear industrial need for cheese ripening enzymes such as proteases, and over the years several commercial protease preparation have been introduced into the market. An overview of available commercial products is given in several papers (Wilkinson, van den Berg & Law, Bulletin of the IDF (2002) 371, 16-19; Kilcawley, Wilkinson & Fox, Food Biotechnol (2002) 16, 29-55; Kilcawley, Wilkinson & Fox, Enzyme Microb. Technol. (2002) 31, 310-320). Examples include enzyme preparations derived from fungal species (including but not limited to Bioprotease P Conc and Bioprotease A conc from Quest, The Netherlands, Protease M & Protease A and Acid protease A from Amano, Promod 215 from Biocatalysts, Sternzyme B5026 from Stern, Flavourzyme MG/A from Novozymes, Denmark; Accelerzyme CPG from DSM, The Netherlands) and bacterial species (including but not limited to Protamex and Neutrase from Novozymes, Denmark, Protease N from Amano, Promod 24P and 24L from Biocatalysts, Protease B500 from DSM, The Netherlands, and Protease 200L from Rhodia Foods, France). The proteases vary considerably in composition with respect to presence of specific proteases and/or the ratio in which these proteases occur in a specific product. The papers by Kilcawley, Wilkinson and Fox, referred to above, clearly shows that most commercial protease products are a mixture of endo- and exo-peptidase activities. Several products are developed to contain only exo-peptidase activity. For food applications, these are invariably amino-peptidases, and examples include DBS50 and DBP20 (from Rhodia, France), Corolase LAP (from Rohm, Germany), Flavourzyme MG/A (from Novozymes, Denmark), Accellerzyme AP and Accelerzyme CPG (from DSM, The Netherlands) and Peptidase R (from Amano, Japan). The amino peptidases and carboxy peptidases are developed and selected for the release of amino acids that are important precursors of cheese flavour such as leucine, phenylalanine and valine. Several patent applications (e.g. WO96/38549) describe the preparation and use of amino peptidases, free from endo-proteases, which can be used to accelerate cheese ripening. Patent application WO2005/074695 describes the use of a particular carboxy peptidase, derived from *Aspergillus niger,* to enhance cheese flavour formation in cheeses containing a bacterial micro flora. The carboxy peptidase is derived from *Aspergillus niger* and is coded in literature as CPD-1. The enzyme has been described (Dal Degan, Ribadeau-dumas & Breddam, Appl. Environ. Microbial (1992) 58, 2144-2152) and sequenced (Svendsen & Dal Degan, Bioch. Biophys. Acta (1998) 1387, 369-377).

Protease addition for cheese ripening can be done in various stages of cheese preparation. Preferably, the enzymes are added to the cheese milk prior to or together with the addition of the coagulant (e.g. chymosin). Addition at this point ensures a homogenous distribution of the enzymes throughout the cheese. Alternatively, the enzymes can be added at a later stage, e.g. during the salting stage in Cheddar making, but this introduces the risk of inhomogeneous enzyme distribution in the cheese and formation of so-called hot spots. For that reason, addition of the enzymes to the cheese milk is preferred. A disadvantage is that the majority of the enzyme (60-90%) is often not incorporated in the cheese curd, and is discarded in the whey fraction where it can give rise to unwanted (proteolysis) that makes the whey less or not suited for further applications. Especially endo-proteases with significant activity at pH5-7 could cause such unwanted side-activities, but also amino peptidase that often have optimal activity in this pH range may give rise to formation of e.g. unwanted flavours. Another potential problem of especially endo-protease addition to the cheese milk is that they interfere with the coagulation process, giving rise to a-specific hydrolysis leading to reduction of cheese yield. Also amino-peptidases can cause yield-losses since they usually are well active at pH6-7, the usual pH range of cheese making. Proteases that are not or almost not active at these pH values during cheese making, but which become active in the cheese are preferred because they will not interfere with the cheese making process and will not cause unwanted reactions in the whey.

Patent application WO2005/074695 describes the cloning and production of a carboxy peptidase and its use in the acceleration of cheese ripening. The authors show that the addition of a single carboxy peptidase to the cheese milk results in an accelerated ripening of the cheese produced with this milk. This is demonstrated by the application of the enzyme in cheeses based on a bacterial micro-flora; no examples were shown for cheeses which contain a mixed flora of bacteria and fungi.

AN1983-13016K (DATABASE WPI Week 198306 Thomson Scientific, London, GB) describes a purification method of acidic carboxypeptidase produced by a microorganism belonging to *Penicillium.* Cheese, soy sauce and other food are produced using exopeptidase and endopeptidase. In the production of these foods, the role of exopeptidase in decomposition of bitter peptide is known to be important.

GRIPON ET AL ("The proteolytic system of Penicillium roqueforti",BIOCHIMIE, MASSON, PARIS, FR, vol. 59, no. 8-9, 28 November 1977 (1977-11-28), pages 679-686) describe the isolation of an alkaline aminopeptidase from the culture medium of *Penicillium roqueforti.*

BOUTROU ET AL ("Enhanced proteolytic activities of Geotrichum candidum and Penicillium camembertii in mixed culture",ENZYME AND MICROBIAL TECHNOLOGY, STONEHAM, MA, US, vol. 39, no. 2, 26 June 2006 (2006-06-26), pages 325-331*)* describe the proteolytic activities involved in the synergistic effect of pure and mixed cultures of *Geotricium candidum* and *Pencillium camemertii* on Camembert juice.

WO 2005/117595 describes the use of a proline-specific endoprotease to improve the flavour of baked cereal products. This publication further describes compositions comprising a proline-specific endoprotease and an exopeptidase and dough comprising such a composition suitable for improving flavour in baked cereal products.

### Detailed description of the invention

The present invention relates to the use of a carboxypeptidase preparation in a method to accelerate the growth of a fungus or mould in a fermented food.

One advantage of the use according to the invention is that mould growth is observed in the fermented food much earlier than when no carboxypeptidase preparation is used. In one embodiment, mould growth is observed one and a half times, twice or three times as fast as usual, i.e. when no carboxypeptidase preparation is used.

Another advantage of the use according to the invention is that a more even and consistent distribution of mould growth is observed.

Yet another advantage of the use according to the invention is that a lower level of bitterness and a more rounded flavour profile is obtained.

The fungus is preferably a species from the genus Penicillium, which includes P. roqueforti and related subspecies.

Suitable examples of fermented food which may be used in the method include beer, sausages and fermented dairy products. The fermented food produced by a process of the invention is a cheese. More preferably, the fermented food is a blue cheese. Well-known examples of blue cheese include Stilton cheese.

The carboxypeptidase preparation addition for cheese ripening can be done in various stages of cheese preparation. In the process of the invention, the carboxypeptidase preparation is added to the cheese milk. This may be prior to or together with the addition of the coagulant. Addition to the cheese milk promotes a homogenous distribution of the enzymes throughout the cheese. Alternatively, the enzymes can be added at a later stage, but this introduces the risk of inhomogeneous enzyme distribution in the cheese and formation of so-called hot spots.

In a preferred embodiment, the carboxypeptidase preparation is free from endoprotease activity, and is able to at least release amino acids that are important for cheese flavour formation, such as leucine, phenylalanine, valine and methionine. The carboxypeptidase preparation is added at activity levels between 1 and 2500 CPG/g substrate (e.g. cheese milk), preferably 1-250 CPG/g substrate or more preferably 1-25 CPG/g substrate. CPG-units are as defined in Example 1 of WO2005/074695. Protease activity is measured by the hydrolysis of a casein (6 g/L assay solution) at pH6.0, 4°C for 1 hour. 1 PU is the amount of enzyme that it produces, in one minute, a (TCA-soluble) hydrolysate, which (280 nm) absorbance is equal to a tyrosin solution of 1 µM).

The carboxypeptidase preparation is defined as free of endo-protease activity when the ratio of endo-protease activity (PU) / carboxypeptidase activity (CPG) in the preparation is less than 0.01, preferably less than 0.001 and most preferably less than 0.0005. The carboxypeptidase preparation preferably shows broad spectrum carboxypeptidase activity through which it is able to release the majority of the natural amino acids from peptides or proteins. Broad spectrum carboxypeptidase activity is defined as activity which releases at least 80% of the natural amino acids in amounts detectable by the method as described in example 3 of WO2005/074695.

In a preferred embodiment of the invention, the carboxypeptidase activity of the carboxypeptidase preparation is for at least 90% caused by a single enzyme, whereby carboxypeptidase activity is measured as described in example 1 of WO2005/074695. In another preferred embodiment, combinations of carboxypeptidases are used.

In a preferred embodiment of the invention, the carboxypeptidase activity is from a purified carboxypeptidase CPD I (PEPG) from *Aspergillus niger.*

In the current application we demonstrate that the addition of a carboxypeptidase to the cheese milk resulted in accelerated fungal growth in e.g. blue veined cheese while maintaining a well balanced cheese flavour. It is known that fungi produce a broad spectrum of endo- and exo-proteases during growth in cheese (J.C. Gripon in Encyclopaedia of dairy Sciences (2003), Roginsky et al, eds, Academic Press, pp 410-406) which liberates amino acids and small peptides as nutrients for the growing fungus. Considering this abundant protease production by the fungus, it is surprising that the addition of the single carboxy peptidase leads to significant fungal growth.

It is also surprising that the accelerated fungal growth does not result in flavour defects. Excessive fungal growth is known to give flavour defects (e.g. bitterness) in cheese due to an imbalance in the proteolytic systems present in cheese (coagulant, bacterial proteases etc. as described previously in this text) and the fungal proteolytic system. Accelerated fungal growth is expected to give an imbalance in the proteolytic processes and therefore flavour defects. It is therefore surprising that accelerated fungal growth can be achieved without creating flavour defects. The invention provides a method for obtaining accelerated growth of a fungus in a cheese comprising adding a carboxypeptidase preparation to the cheese milk during the production of said cheese. Preferably, the obtained fermented food is compared in respect of the fungal growth to a fermented food produced with the same method but without the addition of (i.e. in the absence of) a carboxypeptidase preparation. In yet another preferred embodiment the fungal growth is monitored at least once, for example by visual inspection, and such an embodiment preferably further comprises comparing fungal growth in a fermented food produced in the presence of a carboxypeptidase preparation compared to a fermented food produced in the absence of a carboxypeptidase preparation.

The invention is defined by the appended claims:
1. A process for the accelerated growth of a fungus in a cheese wherein a carboxypeptidase preparation is used and wherein the carboxypeptidase preparation is added to cheese milk.
2. A process according to claim 1 wherein the fungus is a species from the genus Penicillium.
3. A process according to claims 1-2 wherein the cheese is a blue cheese.
4. A process according to claims 1-3 wherein the carboxypeptidase activity of the carboxypeptidase preparation is for at least 90% caused by a single enzyme.
5. A process according to claims 1-4 wherein the ratio of endoprotease activity (PU) and carboxypeptidase activity (CPG) in the carboxypeptidase preparation is less than 001, preferably less than 0001 and most preferably less than 00005
6. A process according to claims 1-5, wherein the carboxypeptidase is CPD-1

### Example 1

### Use of a carboxy peptidase in preparation of Stilton cheese

Blue Stilton was produced using a standard manufacturing protocol. Starter culture (0.01%) and spores of Penicillium roqueforte were added to the cheese milk (29-31 °C), followed after 20 minutes by addition of the coagulant. In this case Accelerzyme CPG (DSM, The Netherlands) was used, this was added together with the coagulant (10 mL / 100 L cheese milk). The curd was set in 60-90 minutes and then cut. The curd was allowed to settle for 60 minutes, drained and left overnight at ambient temperature. The curd was subsequently put through a peg mill, salted (3.5% w/w) and drained in porous moulds for 4 days. The curd was than removed from the vats and stored at 10-12 °C. After 6 weeks, the cheese was pierced with stainless steel needles and left for 6 days to start mould growth. Mould growth was monitored by visual inspection. The cheeses containing Accelerzyme CPG showed visible mould growth after 3 days, those without Accelerzyme CPG after 6 days. Cheeses were tasted after 8 weeks by a professional panel. The cheese with Accelerzyme CPG did not show any defects. With Accelerzyme it showed more rapid and more even and consistent distribution of mould growth. This resulted in a lower level of bitterness and more rounded flavour profile for which the panel showed a preference.

### Example 2

### Application Accelerzyme CPG in Danablue cheese

Two cheese vats of semi-hard blue veined cheese were made. Both vats contained 15.000 liters of the same batch of cheese milk. The cheese milk was standardized with skim and full fat pasteurized milk in such a way that the final cheese will get a fat in dry matter of 50-60%.

Vat A and B were both produced according to the standard Danablue cheese making recipe, with this difference that to vat B 1.5 liters of Accelerzyme CPG 900 (DSM, the Netherlands) was added together with the rennet.

The cheese making process of semi hard blue veined cheese comprised the following steps:
- The milk was standardized to the desired level of fat and protein
- The milk was pasteurized in order to eliminate pathogen micro organisms
- The cheese vats were filled with the desired amount of cheese milk, and brought to the required renneting temperature.
- The following ingredients were added to the milk: lactic acid bacteria cultures; mould spores; calcium chloride and rennet.
- The ingredients were agitated sufficiently to let the milk coagulate until a firm gel is formed.
- The gel was cut in curd-cubs and stirred until the cubs have released enough moisture and are firm enough for further processing.
- The curd was separated from the whey and collected in forms to allow the curds to settle and drain off more whey.
- The curd was led to rest in the moulds in order to acidify the curd mass until the curds formed a cheese-block that is strong enough to be handled.
- Holes were pierced in the curd block in order to allow oxygen to enter the cheese at ripening.
- The curd blocks were taken out of the mould and immersed in saturated brine for 12-16 hours, until the final cheese has taken up the desired amount of salt.
- The cheeses were stored for 3 weeks at 10°C; the surface of the cheese was washed, wrapped in vacuum foil and ripened further at 6°C for 9-11 weeks.

At the end of the ripening the cheese should have the right taste, texture and visual mould growth. After 12 weeks of ripening the cheeses of vat B scored good on all three quality aspects, while the cheeses of vat A had a good taste, but a too brittle texture and poor growth. Ripening the cheeses of vat A for another 2 weeks brought them to the same level of quality as the cheeses of vat B, meaning that the total ripening time was reduced with 2 weeks because of the stimulation of mould growth by Accelerzyme CPG.

## Claims

1. A process for the accelerated growth of a fungus in a cheese wherein a carboxypeptidase preparation is used and wherein the carboxypeptidase preparation is added to cheese milk.

2. A process according to claim 1 wherein the fungus is a species from the genus Penicillium.

3. A process according to claims 1-2 wherein the cheese is a blue cheese.

4. A process according to claims 1-3 wherein the carboxypeptidase activity of the carboxypeptidase preparation is for at least 90% caused by a single enzyme.

5. A process according to claims 1-4 wherein the ratio of endoprotease activity (PU) and carboxypeptidase activity (CPG) in the carboxypeptidase preparation is less than 0.01, preferably less than 0.001 and most preferably less than 0.0005

6. A process according to claims 1-5, wherein the carboxypeptidase is CPD-1.

## Patentansprüche

1. Verfahren zum beschleunigten Wachstum eines Pilzes in einem Käse, wobei eine Carboxypeptidasezubereitung verwendet wird und wobei die Carboxypeptidasezubereitung zu Käsemilch zugegeben wird.

2. Verfahren gemäß Anspruch 1, wobei der Pilz eine Spezies aus der Gattung Penicillium ist.

3. Verfahren gemäß Ansprüchen 1-2, wobei der Käse ein Blauschimmelkäse ist.

4. Verfahren gemäß Ansprüchen 1-3, wobei die Carboxypeptidaseaktivität der Carboxypeptidasezubereitung zu wenigstens 90 % von einem einzigen Enzym verursacht ist.

5. Verfahren gemäß Ansprüchen 1-4, wobei das Verhältnis von Endoproteaseaktivität (PU) und Carboxypeptidaseaktivität (CPG) in der Carboxypeptidasezubereitung weniger als 0,01, vorzugsweise weniger als 0,001 und höchst bevorzugt weniger als 0,0005 beträgt.

6. Verfahren gemäß Ansprüchen 1-5, wobei die Carboxypeptidase CPD-1 ist.

## Revendications

1. Procédé pour la croissance accélérée d'un champignon dans un fromage, dans lequel on utilise une préparation de carboxypeptidase et où la préparation de carboxypeptidase est ajoutée au lait de fromagerie.

2. Procédé selon la revendication 1, dans lequel le champignon est issu d'une espèce du genre *Pénicillium.*

3. Procédé selon les revendications 1-2, dans lequel le fromage est un fromage à pâte persillée.

4. Procédé selon les revendications 1-3, dans lequel l'activité de carboxypeptidase de la préparation de carboxypeptidase est, pour au moins 90%, provoquée par une enzyme unique.

5. Procédé selon les revendications 1-4, dans lequel le rapport de l'activité d'endoprotéase (PU) et d'activité de carboxypeptidase (CPG) dans la préparation de carboxypeptidase est inférieur à 0,01, préférablement inférieur à 0,001 et tout préférablement inférieur à 0,0005.

6. Procédé selon les revendications 1-5, dans lequel la carboxypeptidase est CPD-1.
